# EUROPEAN PATENT APPLICATION

(11) **EP 4 722 373 A1**
(43) Date of publication of application: **08.04.2026**
(21) Application number: 24814605.2
(22) Date of filing: 31.05.2024
(51) Int. Cl.: C12N 15/861, A61K 35/761, C12N 7/01

(54) **ADENOVIRAL VECTOR VACCINE, AND PREPARATION METHOD AND USE THEREOF**

(30) Priority: 02.06.2023 CN 202310649077
(71) Applicant: Cansino Biologics Inc., Tianjin 300457 (CN)
(72) Inventor: SHAO, Juan, Tianjin 300457 (CN); CAO, Longlong, Tianjin 300457 (CN); SUI, Xiuwen, Tianjin 300457 (CN); QI, Yuanyuan, Tianjin 300457 (CN); TONG, Tong, Tianjin 300457 (CN); WU, Dan, Tianjin 300457 (CN); XU, Zhijun, Tianjin 300457 (CN); ZHAO, Jiayu, Tianjin 300457 (CN); WEI, Menghan, Tianjin 300457 (CN); MA, Xinjian, Tianjin 300457 (CN); ZHU, Tao, Tianjin 300457 (CN)
(74) Representative: Finnegan Europe LLP
(86) International application number: PCT/CN2024/096745
(87) International publication number: WO 2024/245411

(57) **Abstract**

Provided are a modified recombinant adenoviral vector, and a preparation method and use thereof. The modified adenoviral vector can reduce the negative charge level on the surface of adenovirus particles, so as to reduce interaction between the adenoviral vector and PF4 (platelet factor 4). By administering the modified adenovirus vector, the risk of thrombus can be reduced, vaccine safety and effectiveness are improved, and the vaccine is more suitable for high-risk crowds such as elder people, children and pregnant women. The recombinant adenovirus can be widely used for gene therapy, tumor immunization and/or antiviral vaccination, including the use of initiating a primary immune response in a human or mammal to initiate a reinforced immune response for destroying the tolerance of a host to an autoantigen. The modified recombinant adenovirus can be used for developing a mucosal administration formulation. Compared with injection, the mucosal administration formulation has compliance and also lower dosage, and can generate triple protection effects of humoral immunity, cellular immunity and mucosal immunity.

## Description

### TECHNICAL FIELD

The present disclosure relates to the technical field of vaccines, and in particular to a method for preparing an artificially modified adenovirus vector vaccine and use thereof. More particularly, the present disclosure relates to use of a non-injectable, adenovirus-vectored vaccine with decreased charges on the surface of the adenovirus vector.

### BACKGROUND

Adenoviruses (Ads) are a large family of double-stranded DNA viruses with a non-enveloped icosahedral capsid structure found in amphibians, avians, and mammals. Their good molecular genetic characterization makes adenovirus vectors good candidates for use as vaccine carriers. The production of recombinant adenovirus vectors relies on the use of packaging cells that can complement the adenovirus gene products in functionality. The adenovirus is non-enveloped, icosahedral, and symmetrical, with 252 capsomeres. The icosahedral vertex capsomeres are 12 pentons, each with 2 fibers. Other than the pentons, there are 240 non-vertex capsomeres, known as hexons, carrying primary genus and sub-genus specific antigenic determinants and secondary species specific antigenic determinants. The structural specificity of the hexon protein allows the coding of neutralizing epitopes and thus the participation in immune responses. So far, 101 adenoviruses have been isolated from their hosts.

Currently, two well-characterized human subgroup C adenoviral serotypes (i.e., hAd2 and hAd5) are widely used as the source of viral backbone for most adenovirus vectors used in gene therapies. Replication-deficient human adenovirus vectors have also been tested as vaccine vectors for delivery of various immunogens derived from various infectious agents (e.g., viruses, parasites, or bacterial pathogens) and tumor cells, including tumor-associated antigens (TAAs). With the increasing use of replication-deficient human adenovirus vectors, two related problems arise, i.e., the risk of pre-existing immunity and the generation of replication-competent adenoviruses (RCAs) contamination.

In order to solve the above problems, the emergence of chimpanzee adenovirus vectors or other rare serotypes of human adenovirus vectors has provided a route, but the development of new types of adenovirus vectors has resulted in many safety issues. In the development of SARS-CoV-2 vaccines, AstraZeneca's vaccine uses chimpanzee ChAdOx1 adenovirus as the vector, which is under suspicion of thrombosis. The European Medicines Agency stated that its Pharmacovigilance Risk Assessment Committee believes that abnormal thrombosis with thrombocytopenia should be classified as a very rare adverse effect of AstraZeneca's SARS-CoV-2 vaccine. Later, an article published by a joint research team of Cardiff University, UK, and the United States in Science Advances revealed the mechanism by which AstraZeneca's vaccine may cause thrombosis. The British-American joint research team believes that the main cause of thrombosis is probably the interaction between the adenovirus vector ChAdOx1 used in AstraZeneca's vaccine and PF4 (platelet factor 4), which incorrectly triggers the immune system to release anti-PF4 antibodies and their accumulation in the blood. PF4 is involved in the pathological mechanism of HIT (thrombocytopenia), which has a clinical manifestation similar to that of TTS (thrombocytopenia syndrome). ChAdOx1 may also form a stable complex with PF4, exhibiting high electronegativity. In a Brownian dynamics (BD) simulation, freely diffusing PF4 often contacts the capsid surface of ChAdOx1 between hexons, most commonly at the interface of three hexons. Another adenovirus vector Ad-26 used in Johnson & Johnson's vaccine against SARS-CoV-2 also contacts PF4, but less frequently. As a result, the US Center for Disease Control and Prevention and the Food and Drug Administration recommended that the use of the vaccine should be suspended first. Studies at the University of Nebraska Medical Center showed that the causes of thrombosis caused by Johnson & Johnson's vaccine against SARS-CoV-2 were basically the same as those caused by AstraZeneca's vaccine.

It is clear that although the application of adenovirus has a long history, it is clear that the risks of adenovirus are not sufficiently understood in the current studies, and that adenovirus has unpredictable risks. Based on the problems found in various types of adenovirus vectors, the inventors have focused on modifying adenovirus vectors to solve the safety problems.

### SUMMARY

In order to achieve the above objective, the inventors conducted a number of biological studies on the modification of adenoviruses, and determined the method for modifying adenovirus vectors through a number of experiments. Provided are a recombinant adenovirus and a method for modifying same.

Specifically, provided is a recombinant adenovirus, wherein one or more negatively charged amino acids in the hexon protein of the adenovirus are mutated into neutral amino acids or positively charged amino acids.

The mutation region of the hexon protein of the adenovirus is the hypervariable region (HVR) of the hexon protein of the adenovirus.

The hypervariable region (HVR) of the hexon protein of the adenovirus comprises one or more of HVR1, HVR2, HVR3, HVR4, HVR5, HVR6, and HVR7.

The negatively charged amino acid is aspartic acid and/or glutamic acid.

The neutral amino acid is selected from one or more of asparagine, glutamine, glycine, alanine, leucine, isoleucine, valine, cysteine, methionine, threonine, serine, and phenylalanine.

The neutral amino acid is selected from one or more of valine (V), leucine (L), asparagine (N), glutamine (Q), and isoleucine (I).

The positively charged amino acid is selected from one or more of arginine (R), lysine (K), and histidine (H).

PF4 binds to the hexons of adenovirus. There are a large number of antigenic peaks and dense hydrophilic peak regions in the front segment and the middle segment of the hexon. This region contains a large number of conserved regions with more hydrophobic amino acids, which become key amino acids for maintaining the spatial structural framework of the hexon and are very important for the formation of the secondary structure. The modification of adenoviruses must be based on maintaining a proper structure, ensuring the basic functionality of the adenovirus, and solving the problem of interaction with PF4.

The recombinant adenovirus vector of the present disclosure can be used in gene therapy, antitumor immunization, and/or anti-viral immunization. The recombinant adenovirus vector of the present disclosure can be used in gene delivery. The adenovirus particle can be specifically directed to a target cell of interest, and the adenovirus binds to the specific target cell through capsid-receptor binding or in other ways and delivers the transgene.

The present disclosure further provides a composition comprising the recombinant adenovirus vector engineered by the method described above.

The present disclosure has the following beneficial effects:
1. The present disclosure provides a recombinant adenovirus with modified charges on the surface of the recombinant adenovirus, so as to reduce the probability of interaction with PF4, solve the safety problem of adenoviruses, and reduce the probability of thrombotic events caused by the use of adenoviruses.
2. In one aspect of the present disclosure, the recombinant adenovirus vector of the present disclosure is prepared into a vaccine vector for delivering various immunogens, and the immune effect is superior to that of existing commonly used adenovirus vectors.
3. In one aspect of the present disclosure, the recombinant adenovirus vector of the present disclosure can be used in gene therapy, antitumor immunization, and/or antiviral immunization, and can be used in gene delivery. The adenovirus particle can be specifically directed to a target cell of interest, and the adenovirus binds to the specific target cell through capsid-receptor binding or in other ways and delivers the transgene.
4. In one aspect of the present disclosure, the composition prepared based on the recombinant adenovirus vector of the present disclosure has fewer related adverse effects and higher safety in clinical application.
5. In one aspect of the present disclosure, a transmucosal formulation can be developed using the modified recombinant adenovirus, for example, by inhalational inoculation to simulate the virus infection process and stimulate the immune response. The local adverse effects such as injection site pain and the like caused by intramuscular injection can be avoided, and the nebulized vaccine can finally reach the lung through the respiratory tract by inhalation, thereby inducing respiratory tract mucosal immunity while inducing humoral immunity and cellular immunity. The formulation for transmucosal administration of the present disclosure features good compliance, a lower dose, and triple immune effects of humoral immunity, cellular immunity, and mucosal immunity, as compared to injection.

### BRIEF DESCRIPTION OF THE DRAWINGS

FIG. 1 illustrates the surface charges of chimpanzee adenovirus vector ChAdOx1 with mutations in different HVR regions;
FIG. 2 illustrates the virus titer of chimpanzee adenovirus vector ChAdOx1 with mutations in different HVR regions;
FIG. 3 illustrates the surface charges of chimpanzee adenovirus vector ChAdOx1 with combinatorial mutations in HVR2 and HVR7 regions;
FIG. 4 illustrates the virus titer of chimpanzee adenovirus vector ChAdOx1 with combinatorial mutations in HVR2 and HVR7 regions;
FIG. 5 illustrates the surface charges of chimpanzee adenovirus vector ChAdOx1 with combinatorial mutations (to different neutral amino acids) in HVR2 and HVR7 regions;
FIG. 6 illustrates the virus titer of chimpanzee adenovirus vector ChAdOx1 with combinatorial mutations (to different neutral amino acids) in HVR2 and HVR7 regions;
FIG. 7 illustrates the surface charges of chimpanzee adenovirus vector ChAdOx1 with combinatorial mutations in HVR1/2/5/7 regions; and
FIG. 8 illustrates the virus titer of chimpanzee adenovirus vector ChAdOx1 with combinatorial mutations in HVR1/2/5/7 regions.

### DETAILED DESCRIPTION

Unless otherwise defined, all scientific and technical terms used in the present disclosure have the same meaning as commonly understood by those skilled in the art to which the present disclosure relates.

The term "recombinant" as used herein implies that the virus has been artificially modified, that is, the virus is not a native wild-type adenovirus.

The term "recombinant adenovirus" as used herein is based on the adenovirus as used herein and is, at least in sequence, derived from the wild type. Such a recombinant adenovirus can be achieved by molecular cloning using the wild-type genome or parts thereof as the starting material. Known sequences of the wild-type adenovirus genome may also be used to regenerate (parts of) the genome by DNA synthesis, which can be performed by commercial service companies in the field of DNA synthesis and/or molecular cloning (e.g., GeneArt, Invitrogen, GenScripts, and Eurofins) using conventional procedures.

As used herein, the term "fragment" refers to a sequence having an amino- and/or carboxyl-terminal and/or an internal deletion. It will be appreciated that for the purpose of inducing an immune response and generally for the purpose of vaccination, the protein is not necessarily full-length, nor does it necessarily have all the functions of a wild type, which is also applicable to a fragment of the protein.

As used herein, the term "composition" refers to a vaccine or other composition prepared from a recombinant adenovirus that can express the target gene by using an adenovirus as a vector and recombining the target gene into the adenovirus genome.

As is conventional in the art, the sequences are provided herein in the 5' to 3' direction. A recombinant adenovirus, wherein one or more negatively charged amino acids in the hexon protein of the adenovirus are mutated into neutral amino acids or positively charged amino acids. The mutation region of the hexon protein of the adenovirus is the hypervariable region (HVR) of the hexon protein, comprising one or more of HVR1, HVR2, HVR3, HVR4, HVR5, HVR6, and HVR7; the mutation region of the hypervariable region (HVR) of the hexon protein comprises at least HVR2 and HVR7.

The negatively charged amino acid is aspartic acid and/or glutamic acid.

The neutral amino acid is selected from one or more of asparagine, glutamine, glycine, alanine, leucine, isoleucine, valine, cysteine, methionine, threonine, serine, and phenylalanine, preferably one or more of valine, leucine, asparagine, and glutamine. The positively charged amino acid is selected from one or more of arginine, lysine, and histidine.

At least one negatively charged amino acid in the hypervariable region (HVR) is mutated into a neutral amino acid.

The mutation region of the adenovirus hexon protein is: D144 and/or E159, or an equivalent position thereof, in HVR1; D171, D175, and/or D177, or an equivalent position thereof, in HVR2; D265 or an equivalent position thereof in HVR5; and/or D423, D425, E427, and/or E429, or an equivalent position thereof, in HVR7.

The adenovirus includes simian adenovirus or human adenovirus.

Such adenoviruses include: any one of the seven subgroups A, B, C, D, E, F, and G of simian adenovirus, such as sAd1, sAd10, sAd11, sAd12, sAd13, sAd14, sAd15, sAd16, sAd18, sAd19, sAd2, sAd20, sAd23, sAd25, sAd26, sAd27, sAd28, sAd29, sAd3, sAd30, sAd31, sAd32, sAd33, sAd34, sAd35, sAd37, sAd38, sAd39, sAd4, sAd40, sAd43, sAd44, sAd45, sAd46, sAd47, sAd48, sAd49, sAd6, sAd9, AdY25, AdC8, AdC22, AdC30, AdC37, AdC11, AdC3, AdC17, AdC19, AdC31, AdC20, AdC24, AdC16, AdC26, AdC82, AdC5, AdC7, AdC44, AdC38, AdC43, AdC63, AdC147, AdC73, AdC6, AdC55, AdC83, AdC143, AdC144, AdC145, AdC10, AdC28, AdC9, AdC6
any one of the seven subgroups A, B, C, D, E, F, and G of Rhesus adenovirus, such as RhAd51, RhAd52, RhAd53, RhAd54, RhAd55, RhAd56, RhAd57, RhAd58, RhAd59, RhAd60, RhAd61, RhAd62, RhAd63, RhAd64, RhAd65, RhAd66, RhAd67, CyAd1 any one of the seven subgroups A, B, C, D, E, F, and G of Gorilla adenovirus, such as GoAd B20, GoAd B22, GoAd B21, GoAd B10, GoAd B11, GoAd B12, GoAd B13, GoAd B14, GoAd B19, GoAd E1, GoAd C10, GoAd C11, GoAd C12, GoAd C13, GoAd C14, GoAd C15, GoAd C16, GoAd C17, GoAd C18, GoAd1, GoAd 2;
any one of the seven subgroups A, B, C, D, E, F, and G of baboon adenovirus, such as BaAd 3;
any one of the seven subgroups A, B, C, D, E, F, and G of human adenovirus, such as any one of Ad1, Ad10, Ad11, Ad12, Ad14, Ad15, Ad17, Ad18, Ad19, Ad2, Ad20, Ad21, Ad22, Ad23, Ad25, Ad26, Ad27, Ad28, Ad29, Ad3, Ad30, Ad31, Ad32, Ad33, Ad34, Ad35, Ad36, Ad38, Ad39, Ad4, Ad40, Ad41, Ad42, Ad43, Ad44, Ad45, Ad46, Ad47, Ad48, Ad49, Ad5, Ad50, Ad51, Ad52, Ad53, Ad54, Ad55, Ad56, Ad57, Ad6, Ad60, Ad61, Ad62, Ad63, Ad64, Ad65, Ad66, Ad68, Ad7, Ad89, Ad81, Ad86, Ad8E, AdD8, AdD9, AdD37, AdD10, AdD13, AdD110, AdD109

A method for preparing a recombinant adenovirus, comprising: introducing the recombinant adenovirus into a complementing cell, culturing the cell, and harvesting the virus.

Use of a recombinant adenovirus, comprising gene therapy, antitumor immunization, and/or antiviral immunization.

Use of a recombinant adenovirus, comprising use in inducing a primary immune response, inducing a boosted immune response, or disrupting the tolerance of a host to a self-antigen in a human or mammal.

A composition comprising the recombinant adenovirus and at least one heterologous transgene. The heterologous transgene encapsulates a viral or bacterial antigen. The viral or bacterial antigen is selected from: one or more of HIV, rabies virus, dengue virus, Ebola virus, coronavirus, human papillomavirus, hepatitis C virus, hepatitis B virus, rotavirus, measles virus, respiratory syncytial virus (RSV), varicella-zoster virus (VZV), cytomegalovirus, herpes simplex virus type 2, Epstein-Barr virus, human metapneumovirus, parainfluenza virus, influenza virus, *Trypanosoma cruzi* and *Plasmodium falciparum, Mycobacterium tuberculosis, Streptococcus pneumoniae, Moraxella catarrhalis,* non-typeable *Haemophilus influenzae* (NTHi), *Neisseria meningitidis, Neisseria gonorrhoeae, Staphylococcus aureus, Helicobacter pylori,* and *Chlamydia trachomatis.*

The composition comprises a pharmaceutically acceptable excipient, e.g., one or more of a buffer, a protectant, a stabilizer, a surfactant, an osmotic pressure regulator, an adjuvant, a preservative, and an inactivator. The buffer includes one or more of HEPES, HIS, TRIS, PB, succinic acid, and citric acid; the protectant includes one or more of gelatin, ethanol, ethylenediaminetetraacetic acid (EDTA), ethylenediaminetetraacetic acid disodium salt (EDTA-2Na), and magnesium chloride; the stabilizer includes one or more of sucrose, mannitol, fucose, and maltose; the surfactant includes one or more of Tween, Span, and glycerol; the osmotic pressure regulator includes sodium chloride or is absent; preferably, the excipient component includes one or more of mannitol, sucrose, sodium chloride, magnesium chloride, HEPES, polysorbate 80, and glycerol. The composition can be prepared into a mucosal immune formulation, a humoral immune formulation, a cellular immune formulation, or a skin immune formulation. The mucosal immune formulation is in a liquid dosage form, a solid dosage form, a semi-solid dosage form, a gas dosage form, or an inhalation dosage form. The route of administration of the composition is intravenous injection, intramuscular injection, subcutaneous injection, oral administration, buccal administration, sublingual administration, rectal administration, respiratory administration, or transdermal administration. For the route of administration, the respiratory administration is oral inhalation, nasal inhalation, or aerosol inhalation after nebulization using a nebulizing administration device. The composition is in a unit dose of 0.05-5 mL, preferably 0.1-1 mL.

Use of the composition, comprising use in a medicament, use in delivering a transgene into a host cell, use in inducing a primary immune response or treating or preventing at least one disease in a human or animal, use in boosting an immune response in a human or animal, and use in inducing an immune response disrupting tolerance to a self-antigen in a human or animal.

A method for manufacturing a composition, comprising: providing a nucleic acid of a protein or a fragment thereof of a heterologous gene, propagating the recombinant adenovirus in a host cell, isolating and purifying the recombinant adenovirus, and formulating the recombinant adenovirus into a pharmaceutically acceptable composition.

To further confirm the efficacy of the present disclosure, in some certain examples, a recombinant chimpanzee adenovirus, ChAdOx1 (SEQ. NO. 1), is provided, wherein one or more negatively charged amino acids in the hexon protein of adenovirus ChAdOx1 are mutated into neutral amino acids or positively charged amino acids. The mutations of the negatively charged amino acids in the recombinant chimpanzee adenovirus ChAdOx1 hexon protein are specifically set forth in SEQ. NOs. 2-15

| Sequence | Mutation region | | | | | | | | | |
|---|---|---|---|---|---|---|---|---|---|---|
| | HVR2 | | | | HVR7 | | | | | |
| | D171 | D175 | D177 | D183 | D423 | D425 | E427 | E429 | D433 | D434 |
| SEQ.NO.2 | N | N | / | / | / | / | / | / | / | / |
| SEQ.NO.3 | / | N | N | / | / | / | / | / | / | / |
| SEQ.NO.4 | N | / | / | N | / | / | / | / | / | / |
| SEQ.NO.5 | N | N | N | / | / | / | / | / | / | / |
| SEQ.NO.6 | / | N | N | N | / | / | / | / | / | / |
| SEQ.NO.7 | N | / | N | N | / | / | / | / | / | / |
| SEQ.NO.8 | / | / | / | / | N | N | Q | Q | N | N |
| SEQ.NO.9 | / | / | / | / | N | N | Q | Q | / | / |
| SEQ.NO.10 | / | / | / | / | N | N | / | / | N | N |
| SEQ.NO.11 | / | / | / | / | / | / | Q | Q | N | N |
| SEQ.NO.12 | / | / | / | / | N | N | / | / | N | / |
| SEQ.NO.13 | / | / | / | / | N | N | Q | / | / | / |
| SEQ.NO.14 | / | / | / | / | N | N | Q | Q | N | / |
| SEQ.NO.15 | / | / | / | / | N | N | Q | Q | / | N |

One or more negatively charged amino acids in the hypervariable region (HVR) of the hexon protein, comprising HVR1, HVR2, HVR5, and HVR7, are mutated into neutral amino acids or positively charged amino acids.

The negatively charged amino acid in the hexon protein of the recombinant chimpanzee adenovirus ChAdOx1 is D144 and/or E159 in HVR1; the negatively charged amino acid in the hexon protein is D171, D175, and/or D177 in HVR2; the negatively charged amino acid in the hexon protein is D265 in HVR5; the negatively charged amino acid in the hexon protein is D423, D425, E427, and/or E429 in HVR7. The neutral amino acid after the mutation of the negatively charged amino acid in the hexon protein of the recombinant chimpanzee adenovirus ChAdOx1 is one or more of valine, leucine, asparagine, and glutamine. The positively charged amino acid is selected from one or more of arginine (R), lysine (K), and histidine (H).

The mutation region of the hypervariable region (HVR) of the hexon protein comprises at least HVR2 and HVR7. In some certain examples, the mutations of the negatively charged amino acids in the recombinant chimpanzee adenovirus ChAdOx1 hexon protein are specifically set forth in SEQ. NOs. 16-38

| Sequence | Mutation region | | | | | | |
|---|---|---|---|---|---|---|---|
| | HVR2 | | | HVR7 | | | |
| | D171 | D175 | D177 | D423 | D425 | E427 | E429 |
| SEQ.NO.16 | N | N | N | N | N | Q | Q |
| SEQ.NO.17 | K | K | K | K | K | K | K |
| SEQ.NO.18 | K | N | N | N | N | Q | Q |
| SEQ.NO.19 | K | K | N | N | N | Q | Q |
| SEQ.NO.20 | K | K | K | N | N | Q | Q |
| SEQ.NO.21 | K | K | K | K | N | Q | Q |
| SEQ.NO.22 | K | K | N | N | N | K | K |
| SEQ.NO.23 | K | K | N | K | K | Q | Q |
| SEQ.NO.24 | K | K | K | K | K | Q | Q |
| SEQ.NO.25 | K | K | K | N | N | K | K |
| SEQ.NO.26 | K | N | K | K | K | K | Q |
| SEQ.NO.27 | K | K | K | K | K | K | Q |
| SEQ.NO.28 | K | K | K | K | K | Q | K |
| SEQ.NO.29 | K | K | K | K | N | K | K |
| SEQ.NO.30 | K | K | K | N | K | K | K |
| SEQ.NO.31 | V | V | V | V | V | Q | Q |
| SEQ.NO.32 | T | T | T | T | T | Q | Q |
| SEQ.NO.33 | N | N | N | N | N | L | L |
| SEQ.NO.34 | V | V | V | V | V | L | L |
| SEQ.NO.35 | T | T | T | T | T | L | L |
| SEQ.NO.36 | N | N | N | N | N | I | I |
| SEQ.NO.37 | V | V | V | V | V | I | I |
| SEQ.NO.38 | T | T | T | T | T | I | I |

In some certain examples, provided is a recombinant chimpanzee adenovirus ChAdOx1, wherein the hypervariable region (HVR) of the hexon protein of the adenovirus ChAdOx1 comprises one or more negatively charged amino acids of HVR1, HVR2, HVR5, and HVR7 mutated to positively charged amino acids, SEQ. NOs. 18-30.

| Sequence | Mutation region | | | | | | |
|---|---|---|---|---|---|---|---|
| | HVR2 | | | HVR7 | | | |
| | D171 | D175 | D177 | D423 | D425 | E427 | E429 |
| SEQ.NO.18 | K | N | N | N | N | Q | Q |
| SEQ.NO.19 | K | K | N | N | N | Q | Q |
| SEQ.NO.20 | K | K | K | N | N | Q | Q |
| SEQ.NO.21 | K | K | K | K | N | Q | Q |
| SEQ.NO.22 | K | K | N | N | N | K | K |
| SEQ.NO.23 | K | K | N | K | K | Q | Q |
| SEQ.NO.24 | K | K | K | K | K | Q | Q |
| SEQ.NO.25 | K | K | K | N | N | K | K |
| SEQ.NO.26 | K | N | K | K | K | K | Q |
| SEQ.NO.27 | K | K | K | K | K | K | Q |
| SEQ.NO.28 | K | K | K | K | K | Q | K |
| SEQ.NO.29 | K | K | K | K | N | K | K |
| SEQ.NO.30 | K | K | K | N | K | K | K |

In some certain examples, provided is a recombinant chimpanzee adenovirus ChAdOx1, wherein the mutated region of the hexon protein of the adenovirus ChAdOx1 comprises hypervariable regions HVR1, HVR2, and HVR7 (SEQ. NO. 39), HVR2, HVR5, and HVR7 (SEQ. NO. 40), or HVR1HVR7, HVR2, HVR5, and HVR7 (SEQ. NOs. 41-50).

| Sequence | Mutation site | | | | | | | | | |
|---|---|---|---|---|---|---|---|---|---|---|
| | HVR1 | | HVR2 | | | HVR5 | HVR7 | | | |
| SEQ39 | D144V | E159L | D171N | D175N | D177N | | D423N | D425N | E427Q | E429Q |
| SEQ40 | | | D171N | D175N | D177N | D265N | D423N | D425N | E427Q | E429Q |
| SEQ41 | D144V | E159L | D171N | D175N | D177N | | D423N | D425N | E427Q | E429Q |
| SEQ42 | | | | | | | D423V | D425V | E427L | E429L |
| SEQ43 | | | D171V | D175V | D 177V | | D423N | D425N | E427Q | E429Q |
| SEQ44 | | | | | | | D423V | D425V | E427L | E429L |
| SEQ45 | | | D171T | D175T | D177T | | D423N | D425N | E427I | E429I |
| SEQ46 | | | | | | | D423V | D425V | E427I | E429I |
| SEQ47 | | | D171V | D175V | D 177V | | D423N | D425N | E427I | E429I |
| SEQ48 | | | | | | | D423V | D425V | E427I | E429I |
| SEQ49 | | | D171N | D175N | D177N | | D423N | D425N | E427I | E429I |
| SEQ50 | | | | | | | D423V | D425V | E427I | E429I |

In some certain examples, the collected adenovirus is further purified. The adenovirus purification can be conducted in several steps, including purification, ultrafiltration, diafiltration, or chromatographic separation, and the purification can be conducted by the filtration step to remove cellular debris and other impurities from the cell lysate. The ultrafiltration is used to concentrate the virus solution. Diafiltration or buffer exchange using an ultrafilter is used as the means to remove and exchange salts, sugars, and analogs thereof. Methods for optimizing the conditions for each purification step are known to those of ordinary skill in the art.

A composition, comprising the recombinant chimpanzee adenovirus vector engineered according to the method described above.

Specifically, the viral or bacterial antigen encapsulated by the transgene is selected from: one or more of HIV, rabies virus, dengue virus, Ebola virus, coronavirus, human papillomavirus, hepatitis C virus, hepatitis B virus, rotavirus, measles virus, respiratory syncytial virus (RSV), varicella-zoster virus (VZV), cytomegalovirus, herpes simplex virus type 2, Epstein-Barr virus, human metapneumovirus, parainfluenza virus, influenza virus, *Trypanosoma cruzi* and *Plasmodium falciparum, Mycobacterium tuberculosis, Streptococcus pneumoniae, Moraxella catarrhalis,* non-typeable *Haemophilus influenzae* (NTHi), *Neisseria meningitidis, Neisseria gonorrhoeae, Staphylococcus aureus, Helicobacter pylori,* and *Chlamydia trachomatis.*

The composition described herein comprises a pharmaceutically acceptable excipient, e.g., one or more of a buffer, a protectant, a stabilizer, a surfactant, an osmotic pressure regulator, an adjuvant, a preservative, and an inactivator. The buffer includes one or more of HEPES, HIS, TRIS, PB, succinic acid, and citric acid; the protectant includes one or more of gelatin, ethanol, ethylenediaminetetraacetic acid (EDTA), ethylenediaminetetraacetic acid disodium salt (EDTA-2Na), and magnesium chloride; the stabilizer includes one or more of sucrose, mannitol, fucose, and maltose; the surfactant includes one or more of Tween, Span, and glycerol; the osmotic pressure regulator includes sodium chloride or is absent; preferably, the excipient component includes one or more of mannitol, sucrose, sodium chloride, magnesium chloride, HEPES, polysorbate 80, and glycerol.

The composition described herein can be prepared into a mucosal immune formulation, a humoral immune formulation, a cellular immune formulation, or a skin immune formulation. The mucosal immune formulation is in a liquid dosage form, a solid dosage form, a semi-solid dosage form, a gas dosage form, or an inhalation dosage form.

The route of administration of the composition described herein is intravenous injection, intramuscular injection, subcutaneous injection, oral administration, buccal administration, sublingual administration, rectal administration, respiratory administration, or transdermal administration. The respiratory administration is oral inhalation, nasal inhalation, or aerosol inhalation after nebulization using a nebulizing administration device.

The composition described herein is in a unit dose of 0.05-5 mL, preferably 0.1-1 mL. Use of the composition described herein, comprising use in a medicament, use in delivering a transgene into a host cell, use in inducing a primary immune response or treating or preventing at least one disease in a human or animal, use in boosting an immune response in a human or animal, and use in inducing an immune response disrupting tolerance to a self-antigen in a human or animal.

A method for manufacturing the composition, comprising: providing a nucleic acid of a protein or a fragment thereof of a heterologous gene, propagating the replication-defective chimpanzee recombinant adenovirus in a host cell, isolating and purifying the recombinant adenovirus, and formulating the recombinant adenovirus into a pharmaceutically acceptable composition.

The subject as used herein is preferably a mammal, e.g., a rodent, e.g., a mouse, a cotton rat, or a non-human primate, or a human. The subject is preferably a human subject. The subject may be any age, e.g., from about 1 month old to 100 years old, e.g., from about 2 months old to about 80 years old, e.g., from about 1 month old to about 3 years old, from about 3 years old to about 50 years old, from about 50 years old to about 75 years old, and the like.

The method for further constructing a recombinant adenovirus comprising a viral or bacterial antigen using the recombinant adenovirus described herein comprises a reverse genetics mode, wherein elements such as ori and resistance genes that are not present in a virus genome are removed by digestion with MssI enzyme; the plasmid is linearized and purified by using a commercial purification and recovery kit to remove enzymes and other reaction components; 1-2 µg of the purified linearized plasmids is used to transfect HEK293SF-3F6 cells in a 6-well plate by using a lipo2000 kit; the cells are transferred to a T25 bottle for culture the next day, so as to remove lipo2000 reagents harmful to the cells and provide the cells with a larger growth space; after 7 days, plaques due to viral infection are observed, which are formed due to cell lysis.

The method for further constructing a recombinant adenovirus comprising a viral or bacterial antigen further comprises: optimizing the gene of the viral or bacterial antigen, performing PCR, isolating a product, performing enzyme digestion, and isolating an enzyme-digested fragment. Meanwhile, the plasmid vector is digested with the same endonuclease, and the recombinant vector is recovered. The vector and the backbone plasmid are co-transfected into HEK293SF-3F6 cells for virus rescue. After 10-20 days, plaques due to viral infection are observed, which are formed due to cell lysis.

The technical solutions of the present disclosure will be clearly and completely described below with reference to the examples of the present disclosure, and apparently, the described examples are only exemplary examples of the present disclosure instead of all examples of the present disclosure. Based on the examples of the present disclosure, all other examples obtained by those of ordinary skill in the art without creative efforts shall fall within the claimed scope of the present disclosure.

### Example 1. Chimpanzee adenovirus vector ChAdOx1-HVR mutation (with mutation regions in different HVR regions)

On the basis of the chimpanzee ChAdOx1 adenovirus vector, the following mutations were performed to investigate the effect of mutation regions in different HVR regions on the adenovirus titer and charges of virus purification.

**Table 1. Mutations in different HVR regions of chimpanzee adenovirus vector ChAdOx 1**

| Group | Sequence | HVR2 | | | | HVR7 | | | | | |
|---|---|---|---|---|---|---|---|---|---|---|---|
| | | D171 | D175 | D177 | D183 | D423 | D425 | E427 | E429 | D433 | D434 |
| 1 | SEQ.NO.1 | / | / | / | / | / | / | / | / | / | / |
| 2 | SEQ.NO.2 | N | N | / | / | / | / | / | / | / | / |
| 3 | SEQ.NO.3 | / | N | N | / | / | / | / | / | / | / |
| 4 | SEQ.NO.4 | N | / | / | N | / | / | / | / | / | / |
| 5 | SEQ.NO.5 | N | N | N | / | / | / | / | / | / | / |
| 6 | SEQ.NO.6 | / | N | N | N | / | / | / | / | / | / |
| 7 | SEQ.NO.7 | N | / | N | N | / | / | / | / | / | / |
| 8 | SEQ.NO.8 | / | / | / | / | N | N | Q | Q | N | N |
| 9 | SEQ.NO.9 | / | / | / | / | N | N | Q | Q | / | / |
| 10 | SEQ.NO.10 | / | / | / | / | N | N | / | / | N | N |
| 11 | SEQ.NO.11 | / | / | / | / | / | / | Q | Q | N | N |
| 12 | SEQ.NO.12 | / | / | / | / | N | N | / | / | N | / |
| 13 | SEQ.NO.13 | / | / | / | / | N | N | Q | / | / | / |
| 14 | SEQ.NO.14 | / | / | / | / | N | N | Q | Q | N | / |
| 15 | SEQ.NO.15 | / | / | / | / | N | N | Q | Q | / | N |

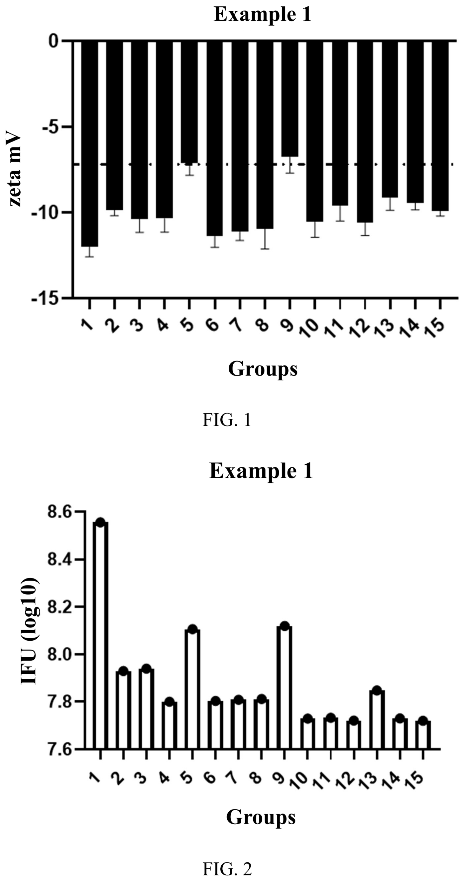

The results, as shown in FIG. 1, demonstrate that ChAdOx1-Hexon_HVR mutation strategy 5 and ChAdOx1-Hexon_HVR mutation strategy 9 exhibited reduced negative charges as compared to the wild-type chimpanzee adenovirus ChAdOx1.

The virus titer results demonstrate that group 1, the wild-type ChAdOx1, exhibited the highest virus titer, followed by group 5 and group 9 - ChAdOx1-Hexon_HVR mutation strategies 5 and 9.

### Example 2. Chimpanzee adenovirus vector ChAdOx1-HVR mutation (with combinatorial mutations in HVR regions)

On the basis of the chimpanzee ChAdOx1 adenovirus vector, the following mutations were performed to investigate the effect of combinatorial mutation regions in HVR2 and HVR7 regions on the adenovirus titer and charges of virus purification.

**Table 2. Chimpanzee adenovirus vector ChAdOx1 with combinatorial mutations in HVR2 and HVR7 regions**

| Group | Sequence | HVR2 | | | HVR7 | | | |
|---|---|---|---|---|---|---|---|---|
| | | D171 | D175 | D177 | D423 | D425 | E427 | E429 |
| 1 | SEQ.NO.16 | N | N | N | N | N | Q | Q |
| 2 | SEQ.NO.17 | K | K | K | K | K | K | K |
| 3 | SEQ.NO.18 | K | N | N | N | N | Q | Q |
| 4 | SEQ.NO.19 | K | K | N | N | N | Q | Q |
| 5 | SEQ.NO.20 | K | K | K | N | N | Q | Q |
| 6 | SEQ.NO.21 | K | K | K | K | N | Q | Q |
| 7 | SEQ.NO.22 | K | K | N | N | N | K | K |
| 8 | SEQ.NO.23 | K | K | N | K | K | Q | Q |
| 9 | SEQ.NO.24 | K | K | K | K | K | Q | Q |
| 10 | SEQ.NO.25 | K | K | K | N | N | K | K |
| 11 | SEQ.NO.26 | K | N | K | K | K | K | Q |
| 12 | SEQ.NO.27 | K | K | K | K | K | K | Q |
| 13 | SEQ.NO.28 | K | K | K | K | K | Q | K |
| 14 | SEQ.NO.29 | K | K | K | K | N | K | K |
| 15 | SEQ.NO.30 | K | K | K | N | K | K | K |

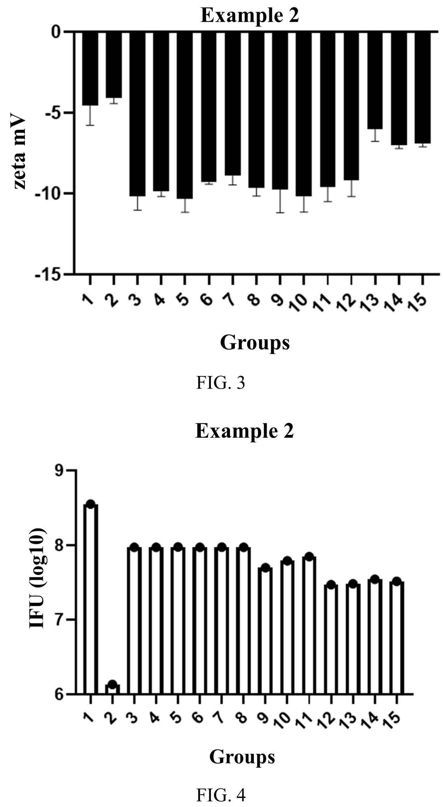

The results, as shown in FIG. 3, demonstrate that groups 1-15, ChAdOx1-Hexon_HVR mutation strategies 16-30, exhibited reduced negative charges as compared to the wild-type chimpanzee adenovirus ChAdOx1.

The virus titer results, as shown in FIG. 4, demonstrate that group 2, ChAdOx1-Hexon_HVR mutation strategy 17, exhibited the lowest virus titer.

### Example 3. Chimpanzee adenovirus vector ChAdOx1-HVR mutation (with combinatorial mutations to different neutral amino acids in HVR2 and HVR7)

On the basis of the chimpanzee ChAdOx1 adenovirus vector, the following mutations were performed to investigate the effect of mutations to different neutral amino acids in HVR2 and HVR7 regions on the adenovirus titer and charges of virus purification.

**Table 3. Chimpanzee adenovirus vector ChAdOx1 with combinatorial mutations (to different neutral amino acids) in HVR2 and HVR7 regions**

| Group | Sequence | HVR2 | | | HVR7 | | | |
|---|---|---|---|---|---|---|---|---|
| | | D171 | D175 | D177 | D423 | D425 | E427 | E429 |
| 1 | SEQ.NO.16 | N | N | N | N | N | Q | Q |
| 2 | SEQ.NO.31 | V | V | V | V | V | Q | Q |
| 3 | SEQ.NO.32 | T | T | T | T | T | Q | Q |
| 4 | SEQ.NO.33 | N | N | N | N | N | L | L |
| 5 | SEQ.NO.34 | V | V | V | V | V | L | L |
| 6 | SEQ.NO.35 | T | T | T | T | T | L | L |
| 7 | SEQ.NO.36 | N | N | N | N | N | I | I |
| 8 | SEQ.NO.37 | V | V | V | V | V | I | I |
| 9 | SEQ.NO.38 | T | T | T | T | T | I | I |

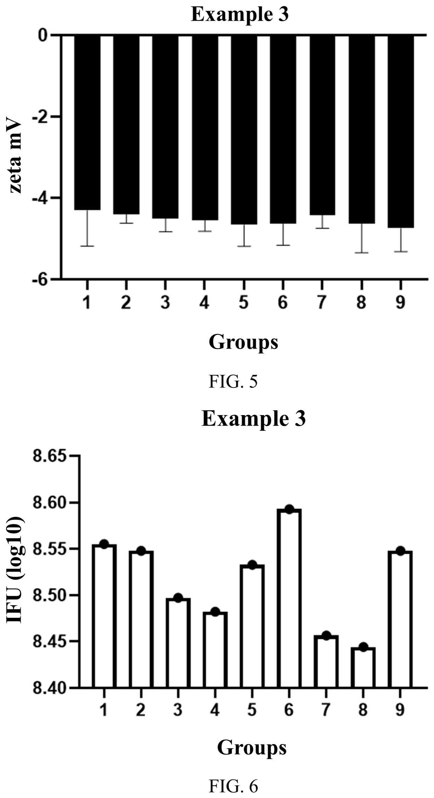

The results, as shown in FIG. 5, demonstrate that groups 1-9, ChAdOx1-Hexon_HVR mutation strategies 31-38, exhibited reduced negative charges as compared to the wild-type chimpanzee adenovirus ChAdOx1.

The virus titer results, as shown in FIG. 6, demonstrate that groups 1-9, ChAdOx1-Hexon_HVR mutation strategies and the wild-type ChAdOx1 (group 1), exhibited similar virus titers, suggesting normal replication.

### Example 4. Chimpanzee adenovirus vector ChAdOx1-HVR mutation (HVR2 and 7 in combination with HVR1 and HVR5)

On the basis of the chimpanzee ChAdOx1 adenovirus vector, the following mutations were performed to investigate the effect of other combinatorial mutations in combination with HVR2 and HVR7 regions on the adenovirus titer, charges of virus purification, and PF4 interaction.

**Table 4. Chimpanzee adenovirus vector ChAdOx1 with combinatorial mutations in HVR1/2/5/7**

| Group | Sequence | Mutation site | | | | | | | | | |
|---|---|---|---|---|---|---|---|---|---|---|---|
| | | HVR1 | | HVR2 | | | HVR5 | HVR7 | | | |
| 1 | SEQ.NO.39 | D144V | E159L | D171N | D175N | D177N | | D423N | D425N | E427Q | E429Q |
| 2 | SEQ.NO.40 | | | D171N | D175N | D177N | D265N | D423N | D425N | E427Q | E429Q |
| 3 | SEQ.NO.41 | D144V | E159L | D171N | D175N | D177N | | D423N | D425N | E427Q | E429Q |
| 4 | SEQ.NO.42 | | | | | | | D423V | D425V | E427L | E429L |
| 5 | SEQ.NO.43 | | | D171V | D 175V | D 177V | | D423N | D425N | E427Q | E429Q |
| 6 | SEQ.NO.44 | | | | | | | D423V | D425V | E427L | E429L |
| 7 | SEQ.NO.45 | | | D171T | D175T | D177T | | D423N | D425N | E427I | E429I |
| 8 | SEQ.NO.46 | | | | | | | D423V | D425V | E427I | E429I |
| 9 | SEQ.NO.47 | | | D171V | D 175V | D 177V | | D423N | D425N | E427I | E429I |
| 10 | SEQ.NO.48 | | | | | | | D423V | D425V | E427I | E429I |
| 11 | SEQ.NO.49 | | | D171N | D175N | D177N | | D423N | D425N | E427I | E429I |
| 12 | SEQ.NO.50 | | | | | | | D423V | D425V | E427I | E429I |

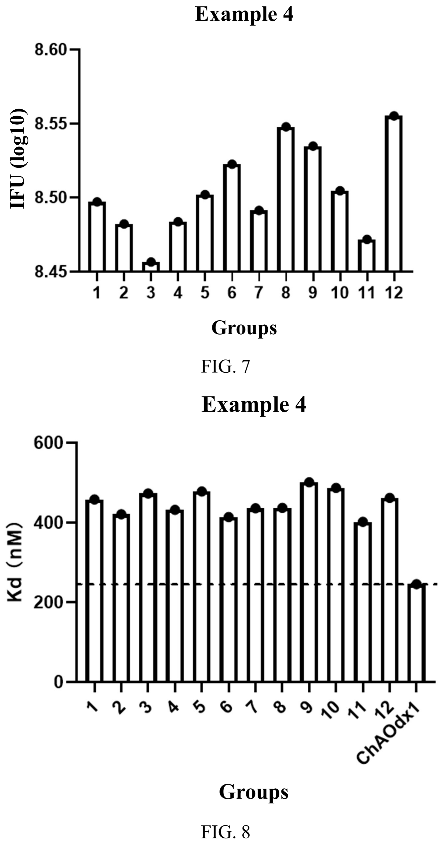

The results, as shown in FIG. 7, demonstrate that groups 1-12, ChAdOx1-Hexon_HVR mutation strategies 39-50, exhibited reduced negative charges as compared to the wild-type chimpanzee adenovirus ChAdOx1.

The results of the PF4 interaction study, as shown in FIG. 8, demonstrate that the dissociation constants of ChAdOx1-Hexon_HVR mutation strategies 39-50 with PF4 were increased, indicating a reduced interaction with PF4.

## Claims

1. A recombinant adenovirus, wherein one or more negatively charged amino acids in the hypervariable region (HVR) of the hexon protein of the adenovirus are mutated into neutral amino acids and/or positively charged amino acids.

2. The recombinant adenovirus according to claim 1, wherein the hypervariable region (HVR) of the hexon protein comprises one or more of HVR1, HVR2, HVR3, HVR4, HVR5, HVR6, and HVR7.

3. The recombinant adenovirus according to claim 1 or 2, wherein the mutation region of the hypervariable region (HVR) of the hexon protein comprises at least HVR2 and/or HVR7.

4. The recombinant adenovirus according to any one of claims 1-3, wherein the negatively charged amino acid is aspartic acid (D) and/or glutamic acid (E); the neutral amino acid is selected from one or more of valine (V), leucine (L), asparagine (N), glutamine (Q), and isoleucine (I); the positively charged amino acid is selected from one or more of arginine (R), lysine (K), and histidine (H).

5. The recombinant adenovirus according to any one of claims 1-4, wherein at least one negatively charged amino acid in the hypervariable region (HVR) is mutated into a neutral amino acid.

6. The recombinant adenovirus according to any one of claims 1-5, wherein the mutation region of the adenovirus hexon protein is: D144 and/or E159, or an equivalent position thereof, in HVR1; D171, D175, and/or D177, or an equivalent position thereof, in HVR2; D265 or an equivalent position thereof in HVR5; and/or D423, D425, E427, and/or E429, or an equivalent position thereof, in HVR7.

7. A method for preparing the recombinant adenovirus according to any one of claims 1-6, comprising: introducing the recombinant adenovirus into a complementing cell, culturing the cell, and harvesting the virus.

8. Use of the recombinant adenovirus according to any one of claims 1-6 in preparing a medicament, comprising gene therapy, antitumor immunization, and/or antiviral immunization.

9. Use of the recombinant adenovirus according to any one of claims 1-6 in preparing a medicament, comprising use in inducing a primary immune response, inducing a boosted immune response, or disrupting the tolerance of a host to a self-antigen in a human or mammal.

10. A composition, comprising the recombinant adenovirus according to any one of claims 1-6, and at least one heterologous transgene, wherein the heterologous transgene encapsulates a viral or bacterial antigen.

11. The composition according to claim 10, wherein the viral or bacterial antigen is selected from: one or more of HIV, rabies virus, dengue virus, Ebola virus, coronavirus, human papillomavirus, hepatitis C virus, hepatitis B virus, rotavirus, measles virus, respiratory syncytial virus (RSV), varicella-zoster virus (VZV), cytomegalovirus, herpes simplex virus type 2, Epstein-Barr virus, human metapneumovirus, parainfluenza virus, influenza virus, *Trypanosoma cruzi* and *Plasmodium falciparum, Mycobacterium tuberculosis, Streptococcus pneumoniae, Moraxella catarrhalis,* non-typeable *Haemophilus influenzae* (NTHi), *Neisseria meningitidis, Neisseria gonorrhoeae, Staphylococcus aureus, Helicobacter pylori,* and *Chlamydia trachomatis.*

12. The composition according to any one of claims 10-11, comprising a pharmaceutically acceptable excipient, wherein preferably, the pharmaceutically acceptable excipient is selected from: one or more of a buffer, a protectant, a stabilizer, a surfactant, an osmotic pressure regulator, an adjuvant, a preservative, and an inactivator.

13. The composition according to any one of claims 10-11, wherein the composition is a mucosal immune formulation, a humoral immune formulation, a cellular immune formulation, or a skin immune formulation.

14. The composition according to claim 13, wherein the mucosal immune formulation is in a liquid dosage form, a solid dosage form, a semi-solid dosage form, a gas dosage form, or an inhalation dosage form.

15. The composition according to any one of claims 10-11, wherein the composition is a formulation for administration; preferably, the formulation for administration is a formulation for intravenous injection, intramuscular injection, subcutaneous injection, oral administration, buccal administration, sublingual administration, rectal administration, respiratory administration, or transdermal administration; preferably, the composition for administration is a formulation for respiratory administration, or is a formulation for oral inhalation, nasal inhalation, or aerosol inhalation after nebulization using a nebulizing administration device.

16. Use of the composition according to any one of claims 10-15 in preparing a medicament for one or more of: gene therapy, antitumor immunization, antiviral immunization, delivering a transgene into a host cell, inducing a primary immune response in a human or animal, boosting an immune response in a human or animal, and inducing an immune response disrupting tolerance to a self-antigen in a human or animal.

17. A method for preparing the composition according to any one of claims 10-15, comprising: providing a nucleic acid of a protein or a fragment thereof of a heterologous gene, propagating the recombinant adenovirus in a host cell, isolating and purifying the recombinant adenovirus, and formulating the recombinant adenovirus into a pharmaceutically acceptable composition.
